(19) Europäisches Patentamt
European Patent Office
Office européen des brevets

(11) Publication number: **0 268 261**
**A1**

(12) # EUROPEAN PATENT APPLICATION

(21) Application number: 87116955.3

(51) Int. Cl.[4]: C07C 148/00

(22) Date of filing: 17.11.87

(30) Priority: 18.11.86 US 932118

(43) Date of publication of application: 25.05.88 Bulletin 88/21

(84) Designated Contracting States: AT BE CH DE ES FR GB GR IT LI LU NL SE

(71) Applicant: PHILLIPS PETROLEUM COMPANY
5th and Keeler
Bartlesville Oklahoma 74004(US)

(72) Inventor: Mark, Harold Wayne
312 E 14
Bartlesville Oklahoma 74003(US)

(74) Representative: Dost, Wolfgang, Dr.rer.nat.,Dipl.-Chem. et al
Patent- & Rechtsanwälte Bardehle . Pagenberg . Dost . Altenburg . Frohwitter . Geissler & Partner Galileiplatz 1 Postfach 86 06 20
D-8000 München 86(DE)

(54) **Process for producing 1-hydrocarbyl mercaptans.**

(57) A process is disclosed for producing 1-hydrocarbyl mercaptans which comprises reacting a 1-hydrocarbyl halide with an alkali hydrogen sulfide or an ammonium hydrogen sulfide in the presence of a quaternary ammonium halide, which serves as a phase transfer catalyst.

EP 0 268 261 A1

# PROCESS FOR PRODUCING 1-HYDROCARBYL MERCAPTANS

The present invention pertains to an improved process for producing 1-hydrocarbyl mercaptans.

It is well known in the art that it is possible to produce 1-hydrocarbyl mercaptans by carrying out an ionic displacement reaction, whereby a 1-hydrocarbyl halogen and an alkali hydrogen sulfide are reacted in a methanolic solution at high temperatures.

However this reaction suffers from having the disadvantage of having to be carried out at high temperatures, generally in the range of 300 - 400°F. In addition, the reaction is quite slow, taking 8 to 10 hours to reach completion and produces only a 60 - 70% yield. Further the by-product of the reaction is a halogenic containing methanol brine which is difficult to dispose of and thus presents environmental concerns.

Thus it is an object of the present invention to provide an improved process for the production of 1-hydrocarbyl mercaptans comprising the use of a phase transfer catalyst. It is a further object of this invention to provide a process for producing 1-hydrocarbyl mercaptans that will not produce a halogenic-containing methanol brine as a by-product.

In accordance with the present invention, I have discovered that reacting a 1-hydrocarbyl halide with an alkali hydrogen sulfide or an ammonium hydrogen sulfide in the presence of a quaternary ammonium halide catalyst results in an improved process for the production of 1-hydrocarbyl mercaptans.

The 1-hydrocarbyl mercaptans which can be produced by this process are those represented by the formula:

$HS - (CH_2)_4 - R$

wherein R can be selected from alkyl, alkenyl, cycloalkyl cycloalkenyl, aryl, or combinations thereof such as alkylaryl, alkenylaryl, cycloalkenylaryl, or cycloalkylaryl having from 8 - 26 carbon atoms.

Representative mercaptans which can be produced by this process can be selected from the group consisting of n-dodecyl mercaptan, n-tridecyl mercaptan, n-tetradecyl mercaptan, n-pentadecyl mercaptan, n-hexadecyl mercaptan, n-heptadecyl mercaptan, n-octadecyl mercaptan, n-nonadecyl mercaptan, and n-eicosanyl mercaptan.

In order to produce the desired 1-hydrocarbyl mercaptans, it is necessary to choose a structurally analogous 1-hydrocarbyl halide. For example, in order to produce n-octadecyl mercaptan it is necessary to use an n-octadecyl halogen as one of the reagents.

Suitable 1-hydrocarbyl halides for use in the present reaction can be represented by the formula:

$X - (CH_2)_4 -R$

wherein R is selected from the group consisting of alkyl, alkenyl, aryl, cycloalkyl, cycloalkenyl, cycloalkylaryl, cycloalkenylaryl or alkylaryl containing from 8 - 26 carbon atoms, and X is a halogen selected from the group consisting of bromine or iodine.

Representative examples can be selected from the group consisting of n-dodecyl bromide, n-dodecyl iodide, n-tridecyl bromide, n-tridecyl iodide, n-tetradecyl bromide, n-tetradecyl iodide, n-pentadecyl bromide, n-pentadecyl iodide, n-hexadecyl bromide, n-hexadecyl iodide, n-heptadecyl bromide, n-heptadecyl iodide, n-octadecyl bromide, n-octadecyl iodide, n-nonadecyl bromide, n-nonadecyl iodide, n-eicosanyl bromide, and n-eicosanyl iodide.

The other reagent utilized in the reaction is an alkali hydrogen sulfide or an ammonium hydrogen sulfide. Suitable alkali hydrogen sulfides can be selected from the grop consisting of sodium hydrogen sulfide, potassium hydrogen sulfide, lithium hydrogen sulfide, or cesium hydrogen sulfide.

It is preferable to utilize an excess of the alkali hydrogen sulfide or the ammonium hydrogen sulfide in the reaction in order to obtain the high yield of final product. Generally, there should be 1.1 -1.7 moles of alkali hydrogen sulfide or ammonium hydrogen sulfide for every mole of 1-hydrocarbyl halide utilized in the reaction. The preferred range is 1.2 - 1.3 moles of alkali hydrogen sulfide or ammonium hydrogen sulfide per mole of 1-hydrocarbyl halide.

The key to the improved process is the use of a quaternary ammonium salt as a phase transfer catalyst which can be represented by the formula:

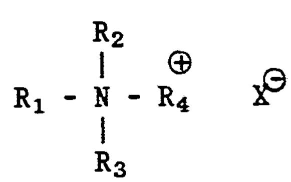

wherein $R_1$, $R_2$, and $R_3$ can be selected from the group consisting of alkyl groups containing from 4 - 12 carbon atoms; $R_4$ is a methyl or ethyl group; and X is a halogen selected from the group consisting of bromine, iodine or chlorine.

Representative examples of suitable phase transfer catalysts are those selected from the group consisting of tributylmethylammonium bromide, tributylmethylammonium chloride, tributylmethylammonium iodide, tributylethylammonium bromide, tributylethylammonium chloride, tributylethylammonium iodide, tripentylmethylammonium bromide, tripentylmethylammonium chloride, tripentylmethylammonium iodide, tripentylethylammonium bromide, tripentylethylammonium chloride, tripentylethylammonium iodide, trihexylmethylammonium bromide, trihexylmethylammonium chloride, trihexylmethylammonium iodide, trihexylethylammonium bromide, trihexylethylammonium chloride, trihexylethylammonium iodide, triheptylmethylammonium bromide, triheptylmethylammonium chloride, triheptylmethylammonium iodide, triheptylethylammonium bromide, triheptylethylammonium chloride, triheptylethylammonium iodide, trioctylmethylammonium bromide, trioctylmethylammonium chloride, trioctylmethylammonium iodide, trioctylethylammonium bromide, trioctylethylammonium chloride, trioctylethylammonium iodide, trinonylmethylammonium bromide, trinonylmethylammonium chloride, trinonylmethylammonium iodide, trinonylethylammonium bromide, trinonylethylammonium chloride, trinonylethylammonium iodide, tridecylmethylammonium bromide, tridecylmethylammonium chloride, tridecylmethylammonium iodide, tridecylethylammonium bromide, tridecylethylammonium chloride, tridecylethylammonium iodide, triundecylmethylammonium bromide, triundecylmethylammonium chloride, triundecylmethylammonium iodide, triundecylethylammonium bromide, triundecylethylammonium chloride, triundecylethylammonium iodide, tridodecylmethylammonium bromide, tridodecylmethylammonium chloride, tridodecylmethylammonium iodide, tridodecylethylammonium bromide, tridodecylethylammonium chloride, and tridodecylethylammonium iodide.

Another suitable ammonium halide phase transfer catalyst can be represented by the formula:

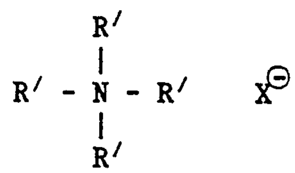

wherein R′ is butyl and $X^{\ominus}$ is a halogen atom selected from the group consisting of bromine, chlorine, or iodine. Examples of suitable quaternary compounds are those from the group consisting of tetrabutylammonium bromide, tetrabutylammonium chloride, or tetrabutylammonium iodide. The catalyst should be present within the reaction system in an amount within the range of 0.5 - 5 mole %, with 1 - 2 mole % being preferred. It is preferred that the reaction be carried out in a polar solvent, such as water. It is also preferred that the reaction is carried out at a temperature in the range of 150 - 250°F, preferably 190 - 210°F. In carrying out the reaction the mixture is generally agitated. This can be accomplished by the use of a mechanical stirrer or any other conventional means. The mercaptans produced by the process of this invention can be readily separated from the reaction mixture by distillation.

The following examples are presented to further illustrate the invention. It is not intended that the invention be unduly limited to the embodiments described therein.

Example I

This example demonstrates the production of 1-hydrocarbyl mercaptans by an ionic displacement reaction carried out in the absence of a quaternary ammonium salt.

A one liter stainless steel autoclave was charged with 190.6 grams (1.7 moles) of a 50 wt.% aqueous solution of NaSH, 333.4 grams (1 mole) of 1-bromooctadecane, 150 ml of 99% methanol and 50 ml of water. The autoclave was closed and heated to 320°F with constant stirring.

Aliquots were withdrawn from the reactor at convenient intervals after the reaction mixture had reached 320°F and were analyzed by vapor phase chromatography to determine the rate at which the alkyl halides were being transformed into alkyl mercaptans. The results are summarized below.

TABLE I

| Elapsed Time (minutes) | Temperature (°F) | $\%C_{18}H_{37}Br$ | $\%C_{18}H_{37}SH$ |
|---|---|---|---|
| 0 | 70 | 100 % | 0 % |
| 15 | 328 | 89.1 | 10.8 |
| 50 | 335 | 55.8 | 43.7 |
| 84 | 334 | 23.4 | 75.6 |
| 125 | 333 | 9.9 | 88.6 |
| 169 | 329 | 4.8 | 93.6 |
| 258 | 329 | 3.2 | 93.7 |
| 345 | 333 | 3.5 | 94.3 |

This data shows that in the absence of a quaternary ammonium salt, it took nearly 6 hours to convert the 1-bromooctadecane into n-octadecyl mercaptan.

Example II

Another sample of n-octadecyl mercaptan was prepared as in Example I (in the absence of a quaternary ammonium salt) except that no aliquots of material were withdrawn from the reactor.

The entire reaction product was collected. The phases were separated by decantation and the organic phase was washed with approximately 200 ml of 120°F water. The resulting mixture was phase separated resulting in a nearly quantitative recovery of crude n-octadecyl mercaptan. The crude product was distilled at 433-462°F at 5 mmHg, to give 196 gm of product, representing a 68% yield.

Example III

The purpose of this example is to demonstrate that the use of quaternary ammonium halides in accordance with the present invention effectively catalyzes the conversion of 1-hydrocarbyl halides into 1-hydrocarbyl mercaptans. A one liter stainless steel autoclave was charged with 190.6 gm (1.7 moles of a 50 weight percent aqueous solution of NaSH, 333.4 gm (1 mole) of 1-bromooctadecane, 4 gm (0.01 mole) of trioctylmethylammonium bromide, and 75 ml of water. The autoclave was closed and heated to approximately 200°F with constant stirring.

Aliquots were withdrawn from the reactor at convenient intervals after the reaction mixture had reached 200°F and were analyzed by vapor phase chromatography to determine the rate at which the alkyl halides were transformed into alkyl mercaptans. The results are summarized below.

TABLE II

| Elapsed Time (minutes) | Temperature (°F) | $\%C_{18}H_{37}Br$ | $\%C_{18}H_{37}SH$ |
|---|---|---|---|
| 0 | 70 | 100 % | 0 % |
| 10 | 211 | 49.6 | 39.1 |
| 19 | 203 | 9.0 | 87.6 |
| 29 | 200 | 1.7 | 96.4 |
| 44 | 201 | -- | 97.9 |

This data shows that in the presence of a quaternary ammonium salt, the conversion of 1-bromooctadecane into n-octadecyl mercaptan took only 44 minutes.

A comparison of the results in Table I, with the results in Table II demonstrates the effectiveness of the present invention. The production of a 1-hydrocarbyl mercaptan in the absence of a phase transfer catalyst took 5½ hours at 320°F. The addition of a phase transfer catalyst decreased the reaction time to approximately 1 hour and the reaction temperature to 200°F.

Example IV

Another sample of n-octadecyl mercaptan was prepared as in Example III, except that no aliquots were removed for vapor phase chromatography. Instead, the entire reaction product was isolated from the reaction mixture by separating the phases via decantation and then washing the organic phase with approximately 200 ml of 120°F water. The phases were again separated resulting in a nearly quantitative recovery of crude n-octadecyl mercaptan. The product was distilled at 433-462°F and 5 mmHg to give 237 grams of product, an 82.4% yield. Thus, not only does the process of the present invention increase the rate of reaction, there is also provided an increase in the yield of final product.

Reasonable variations and modifications can be made in view of the foregoing disclosure without departing from the spirit and scope of the invention.

**Claims**

1. A process for producing a 1-hydrocarbyl mercaptan of the formula
$HS - (CH_2)_4 -R$
wherein R is alkyl, alkenyl, cycloalkyl, cycloalkenyl, aryl or a combination thereof, containing from 8-26 carbon atoms, **characterized by** reacting a 1-hydrocarbyl halide of the formula $R'(CH_2)_4X$ wherein R' has the same meaning as R, and X is a halogen selected from bromine and iodine, with an alkali hydrogen sulfide or ammonium hydrogen sulfide, said alkali being selected from sodium, potassium, lithium and cesium, in the presence of a quaternary ammonium halide of the formula

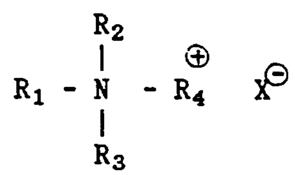

or

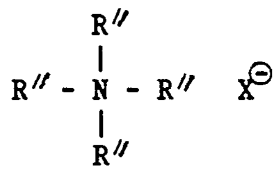

wherein $R_1$, $R_2$ and $R_3$ are independently alkyl groups containing 4-12 carbon atoms, $R_4$ is a methyl or ethyl grop, R″ is butyl, and $X^{\ominus}$ is bromine, iodine or chlorine.

2. The process of claim 1, characterized in that said alkali hydrogen sulfide or ammonium hydrogen sulfide is present in an amount of 1.1 to 1.7 moles for every mole of 1-hydrocarbyl halide present, and said quaternary ammonium halide is present in the quantity of 0.5 to 5 mole %.

3. The process of claim 1 or 2, characterized in that said 1-hydrocarbyl halide is 1-octadecyl bromide, said alkali hydrogen sulfide is sodium hydrogen sulfide and said quaternary ammonium halide is trioctylmethylammonium bromide.

4. The process of any of claims 1 to 3, characterized in that said reaction is carried out at a temperature of 66 to 121°C.

European Patent Office

# EUROPEAN SEARCH REPORT

Application Number

EP 87 11 6955

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 265 737 (CHIMOSA CHIMICA ORGANICA)<br>--- | | C 07 C 148/00 |
| A | US-A-2 404 425 (J.E. BEANBLOSSOM et al.)<br>----- | | |
| | | | TECHNICAL FIELDS SEARCHED (Int. Cl.4) |
| | | | C 07 C 148/00<br>C 07 C 149/00 |

The present search report has been drawn up for all claims

| Place of search | Date of completion of the search | Examiner |
|---|---|---|
| THE HAGUE | 08-02-1988 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)